(19) Europäisches Patentamt / European Patent Office / Office européen des brevets

(11) **EP 1 694 219 B1**

(12) **FASCICULE DE BREVET EUROPEEN**

(45) Date de publication et mention
de la délivrance du brevet:
**20.01.2010 Bulletin 2010/03**

(21) Numéro de dépôt: **04797273.2**

(22) Date de dépôt: **29.11.2004**

(51) Int Cl.:
***A61B 17/16*** *(2006.01)*

(86) Numéro de dépôt international:
**PCT/CH2004/000716**

(87) Numéro de publication internationale:
**WO 2005/051207 (09.06.2005 Gazette 2005/23)**

(54) **Fraise chirurgicale**

Chirurgischer Fräser

Surgery bur

(84) Etats contractants désignés:
**AT BE BG CH CY CZ DE DK EE ES FI FR GB GR
HU IE IS IT LI LU MC NL PL PT RO SE SI SK TR**

(30) Priorité: **28.11.2003 CH 20252003**

(43) Date de publication de la demande:
**30.08.2006 Bulletin 2006/35**

(73) Titulaire: **Favre, Marc-Etienne
1092 Belmont-sur-Lausanne (CH)**

(72) Inventeur: **Favre, Marc-Etienne
1092 Belmont-sur-Lausanne (CH)**

(74) Mandataire: **North, Mathieu
Rue du Seyon 2,
Case Postale 2751
2001 Neuchâtel (CH)**

(56) Documents cités:
**WO-A-02/49516      WO-A-98/31291
FR-A- 1 031 888      FR-A- 1 041 311**

**EP 1 694 219 B1**

## Description

Domaine technique

L'objet de la présente invention est une fraise chirurgicale, et notamment une fraise à cotyle, aussi appelée alésoir « acétabulaire », c'est-à-dire destinée à creuser l'os de la hanche pour y placer une prothèse.

Technique antérieure

En matière médicale, et notamment dans le domaine des fraises à cotyle, on connaît des fraises en forme de calotte hémisphérique, présentant des ouvertures et des arêtes de coupe, analogues à celles des râpes à fromage, et qui sont formées par étampage, découpage et repoussage. On trouve des exemples de telles fraises à tête hémisphérique dans les documents CH 692600, CH 690021, FR 2281095, US 4811632, ou encore US 5100267.

Les fraises du type susmentionné sont relativement chères à fabriquer, en raison notamment des diverses opérations impliquées par cette fabrication.

Dans le domaine médical, et notamment dans celui des fraises à cotyle, le prix de revient empêche les utilisateurs de n'utiliser leurs fraises qu'une seule fois, alors qu'une utilisation unique serait nettement préférable en raison des risques septiques.

Le document US 5100267 décrit une fraise à cotyle à calotte hémisphérique à usage unique. Afin de réduire son prix, la fraise à cotyle décrite dans ce document est pourvue d'un raccord en polymère, facile à fabriquer et bon marché. Néanmoins, la calotte hémisphérique, en acier inoxydable, doit être fabriquée selon les méthodes classiques. C'est dire qu'elle reste chère. De plus, la présence de matière plastique présente souvent des risques, car les matériaux de ce type, fort tendres, peuvent aisément laisser des particules dans les corps, ce qui est souvent mal toléré par le patient.

Les gens du métier continuent donc à utiliser des fraises à cotyle, en général dépourvues de matière plastique, d'un prix élevé, qu'ils doivent stériliser après chaque usage, ce qui entraîne des frais non négligeables.

D'autre part, la construction des fraises est massive, de sorte que la fraise ne présente pas d'interstices qui permettraient à l'utilisateur de voir le fond de la cavité que creuse la fraise. L'utilisateur n'est donc guère en mesure de vérifier la progression du fraisage en cours d'opération, ni l'état des parois de la cavité fraisée. Cela constitue un inconvénient sérieux surtout dans le domaine médical.

D'autres fraises présentent des arêtes de coupe, disposées radialement, en étoile, à partir de l'axe de rotation, et qui rayonnent vers l'extérieur. On trouve de tels exemples dans les brevets FR1041311, FR1031888, US3,702,611 ou US4,621637. Dans ces exemples, les lames sont rapportées, c'est-à-dire fabriquées indépendamment, puis fixées dans la tête, qui présente à cette fin des logements. Des moyens de fixation sont nécessaires (comme des vis ou des tenons), ce qui complique le dispositif

Ce genre de fraise présente les mêmes inconvénients mentionnés plus haut, et notamment la difficulté que rencontre le chirurgien à voir le fond de la cavité que creuse la fraise. Par ailleurs, de telles fraises demeurent chères à fabriquer.

La demande PCT publiée sous WO98/31291 montre une fraise à cotyle de construction relativement simple, dans laquelle des lames plates sont découpées en forme d'arc de cercle dans une feuille métallique et sont assemblées les unes aux autres par des fentes pratiquées dans les lames elles-mêmes. Les lames se croisent à angle droit et s'inscrivent dans une demi-sphère. Pour les fixer sur un outil et les maintenir ensemble, les lames s'insèrent par des tenons placés aux deux bouts de chaque lame dans des trous pratiqués dans une plaque de base. Cette plaque présente elle-même une ouverture centrale qui permet de la fixer sur un outil rotatif

L'inconvénient de la visibilité réduite demeure dans ce modèle de fraise, du fait de la présence de la plaque de base. On peut d'ailleurs se demander si la rigidité de la fraise est suffisante.

La demande PCT publiée sous No WO02/49516 montre un autre alésoir chirurgical formé de lames découpées dans des plaques métalliques et assemblées entre elles par des fentes. Les lames sont au nombre de deux seulement, et se rejoignent sur l'axe de rotation, les fentes d'assemblage étant également sur cet axe. Le préambule de la revendication 1 est basé sur cet état de la technique.

Dans cet alésoir, la visibilité est nettement meilleure, le chirurgien étant à même de voir entre les lames le fond de la cavité que creuse l'alésoir.

L'alésoir en question n'est cependant pas extrêmement rigide, malgré l'affirmation du déposant. L'efficacité du fraisage se ressent d'ailleurs probablement du fait que les lames sont au nombre de deux seulement.

La présente invention propose de fournir une fraise qui soit composée de lames assemblées entre elles par des fentes dont l'axe de symétrie longitudinal coïncide avec l'axe de rotation, ces lames étant au nombre d'au moins quatre. Une telle fraise, facile à fabriquer, sera plus rigide et plus efficace dans la coupe que les alésoirs connus, tout en garantissant une visibilité optimale en cours d'opération. Elle est de plus peu onéreuse à fabriquer, les lames étant toutes découpées dans des feuilles métalliques.

Exposé de l'invention

Généralement, la fraise chirurgicale objet de l'invention est formée de plusieurs lames plates assemblées entre elles au moyen de fentes dont l'axe de symétrie longitudinal coïncide avec l'axe de rotation de la fraise; la fraise comprend au moins quatre lames; une ou plusieurs des fentes desdites lames, ou un ou plusieurs secteurs desdites fentes, ont une largeur différente de celle d'autres fentes et/ou secteurs de fentes.

Dans la forme préférée d'exécution de l'invention, la fraise comprend quatre lames dont la première présente au moins une fente qui part du bord inférieur de ladite lame; la deuxième lame présente au moins une fente partant du sommet de la lame, et chacune desdites fentes a, au moins sur un secteur de sa longueur, une largeur correspondant à l'épaisseur de la lame dans laquelle est ménagée l'autre fente; la troisième lame présente au moins une fente supérieure partant du sommet de la lame, et au moins une fente inférieure partant du bord inférieur de la lame, la largeur de la fente supérieure étant plus grande que la largeur de la fente inférieure; la quatrième lame présente au moins une fente partant du sommet de la lame, cette fente comprenant un secteur extérieur et un secteur intérieur, le secteur extérieur étant plus large que le secteur intérieur; de plus, la largeur de la fente inférieure de la troisième lame correspond à l'épaisseur de la quatrième lame; la largeur du secteur intérieur de la fente de la quatrième lame correspond à l'épaisseur de la troisième lame; la largeur de la fente supérieure de la troisième lame et la largeur du secteur extérieur de la fente de la quatrième lame sont les mêmes et sont dimensionnées de telle façon que, une fois emboîtées l'une dans l'autre, les deux premières lames puissent être insérées dans ladite fente supérieure de la troisième lame et dans le secteur extérieur de la quatrième lame, les troisième et quatrième lames étant elles-mêmes emboîtées l'une dans l'autre.

Dans une deuxième forme d'exécution, dans laquelle il est plus aisé d'obtenir des lames qui ont toutes la même hauteur (en suivant l'axe de rotation de la fraise), forme d'exécution qui est applicable aussi bien à la forme générale qu'à la première forme d'exécution particulière et préférée décrite plus haut, la fente de la première lame de la fraise présente deux secteurs de largeurs différentes, à savoir un secteur extérieur, proche du bord inférieur de la lame, plus large que le secteur intérieur, plus proche du sommet de la lame.

Dans une troisième forme d'exécution, qui vise au même but que la deuxième, la deuxième lame de la fraise présente au moins une fente inférieure partant de son bord inférieur, et cette fente inférieure est plus large que la fente qui part du sommet de la lame.

Dans une quatrième forme d'exécution, applicable aux précédentes, la fraise comprend au moins un élément raidisseur qui encercle partiellement et tient ensemble les lames composant la fraise.

Dans une cinquième forme d'exécution, applicable aux précédentes, l'élément raidisseur est une douille cylindrique.

Dans une sixième forme d'exécution, applicable aux précédentes, et qui est la forme d'exécution préférée, l'élément raidisseur est un anneau plat présentant des encoches dans lesquelles viennent s'insérer les lames, le plan dudit anneau étant perpendiculaire à l'axe de rotation de la fraise.

Chaque lame composant la fraise comprend de préférence au moins une fente dont l'axe de symétrie longitudinal coïncide avec l'axe de rotation de la fraise, une partie tranchante arrondie, de préférence d'une forme sensiblement semi-circulaire, et une partie inférieure plus étroite que la partie tranchante.

Dans une forme d'exécution particulière, utilisable dans une fraise décrite plus haut où les lames peuvent plus aisément être de même hauteur (deuxième forme d'exécution mentionnée), au moins une lame comprend une ou plusieurs fentes dont au moins une présente des secteurs de largeurs différentes.

Dans une forme d'exécution particulière d'une telle lame, celle-ci comprend une fente partant du sommet de la partie tranchante et une fente inférieure partant du bord inférieur, la largeur de la fente supérieure étant différente de la largeur de la fente inférieure.

Dans une forme d'exécution encore plus particulière d'une telle lame, ladite fente supérieure ou inférieure comprend au moins un secteur extérieur et un secteur intérieur, la largeur du secteur extérieur étant plus grande que la largeur du secteur intérieur.

Description sommaire des dessins

La figure 1 est une vue éclatée en perspective cavalière d'une fraise selon l'invention dans laquelle la fraise compte quatre lames.

La figure 2 est une vue en perspective cavalière d'une fraise dans la même forme d'exécution que celle de la figure 1, les éléments étant assemblés pour former la fraise.

La figure 3 est une coupe longitudinale d'une fraise dans la forme d'exécution de la figure 2.

La figure 4 est une vue de dessus d'une fraise dans la forme d'exécution des figures 2 et 3.

La figure 5 est une vue en perspective cavalière d'une fraise dans une forme d'exécution légèrement différente de celle des figures 1 à 4, dans laquelle une douille de plus faible hauteur assure la rigidité de l'ensemble formé par les lames assemblées.

La figure 6 est une vue en coupe longitudinale d'une fraise selon l'invention dans une forme d'exécution particulière, dans laquelle une seconde douille assure la rigidité extérieure de l'ensemble formé par les lames.

La figure 7 est une vue agrandie du centre de la figure 4, montrant le croisement des lames entre elles et les dimensions relatives des fentes ménagées dans les lames pour leur insertion les unes dans les autres.

La figure 8 est une vue de face d'une première lame pour une fraise selon l'invention, dans la deuxième forme d'exécution mentionnée.

La figure 9 est une vue de face d'une deuxième lame pour une fraise selon l'invention, dans la troisième forme d'exécution mentionnée.

La figure 10 est une vue de face d'une troisième lame pour une fraise selon l'invention, utilisable dans les autres formes d'exécution mentionnées et conforme notamment à la neuvième.

La figure 11 est une vue de face d'une quatrième lame pour une fraise selon l'invention, utilisable dans les autres formes d'exécution mentionnées et notamment à la deuxième.

La figure 12 est une vue de face d'un anneau pour une fraise selon l'invention, destiné à assurer la rigidité de la fraise une fois assemblée, selon la sixième forme d'exécution mentionnée.

La figure 13 est une vue en perspective cavalière d'une fraise résultant de l'assemblage des lames représentées aux figures 8 à 11 et de l'anneau plat montré à la figure 12.

Meilleure manière de réaliser l'invention

Une caractéristique importante de l'invention réside dans le fait que les lames peuvent être découpées dans une feuille métallique. Par « feuille métallique », on n'entend pas des feuilles de métaux durs, du type des carbures, mais seulement des feuilles de métaux qui peuvent être étampés, comme l'acier inoxydable. Les lames 1, 2, 3 et 4 de la fraise selon l'invention sont découpées dans une feuille de métal ; dans la forme préférée de l'invention, les lames sont découpées par étampage. Cette manière de faire présente l'avantage d'un prix très bas. Le découpage laser ou par électro-érosion est aussi possible, mais d'un prix moins favorable.
De préférence, l'opération d'étampage par laquelle la lame est découpée donnera également, et dans une même opération, son tranchant au fil 18 de la lame. Il est cependant évidemment possible d'obtenir le tranchant par une opération classique d'affûtage à la meule.
De même, et de préférence, le découpage de dents 16 dans le tranchant de la lame, ainsi que l'inclinaison éventuelle desdites lames par rapport au plan de la lame, peuvent être obtenus par étampage, et dans une seule et même opération. L'expérience montre cependant qu'une telle inclinaison n'est pas indispensable.
Dans la forme d'exécution préférée de l'invention, la fraise est composée de quatre lames 1, 2, 3 et 4.

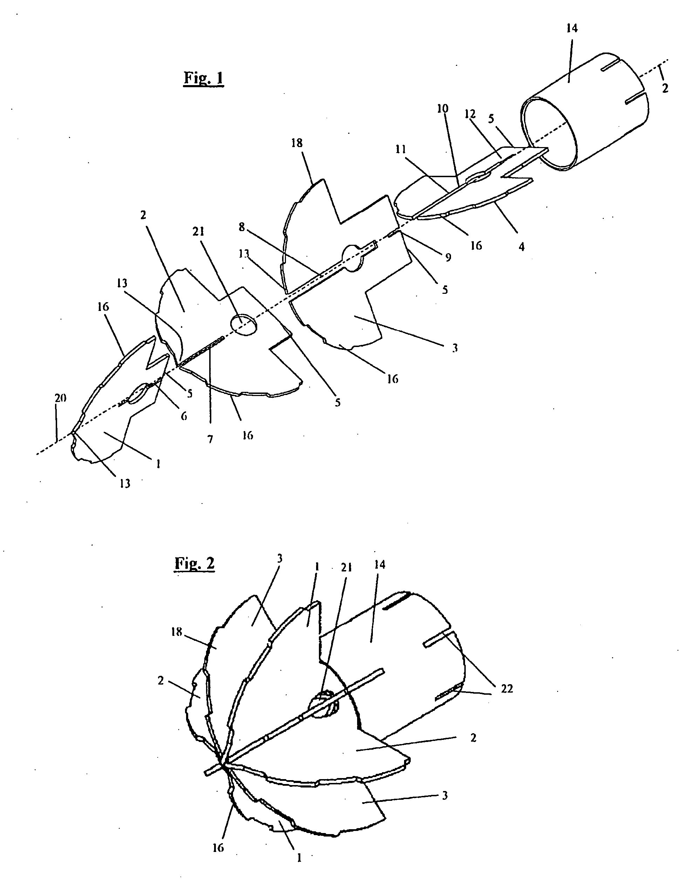

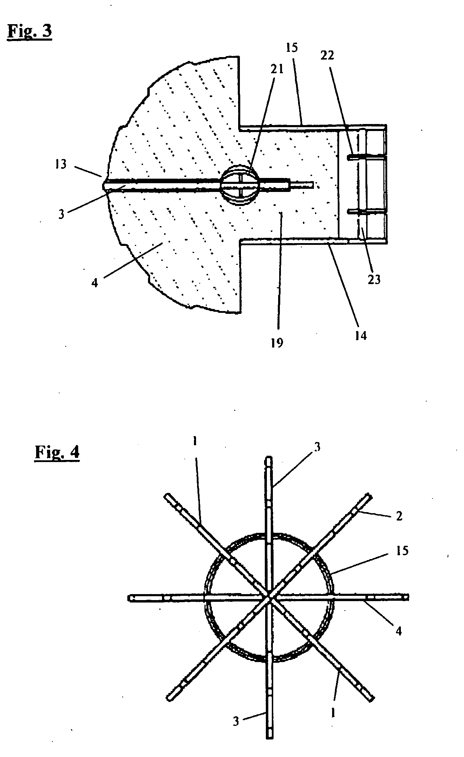

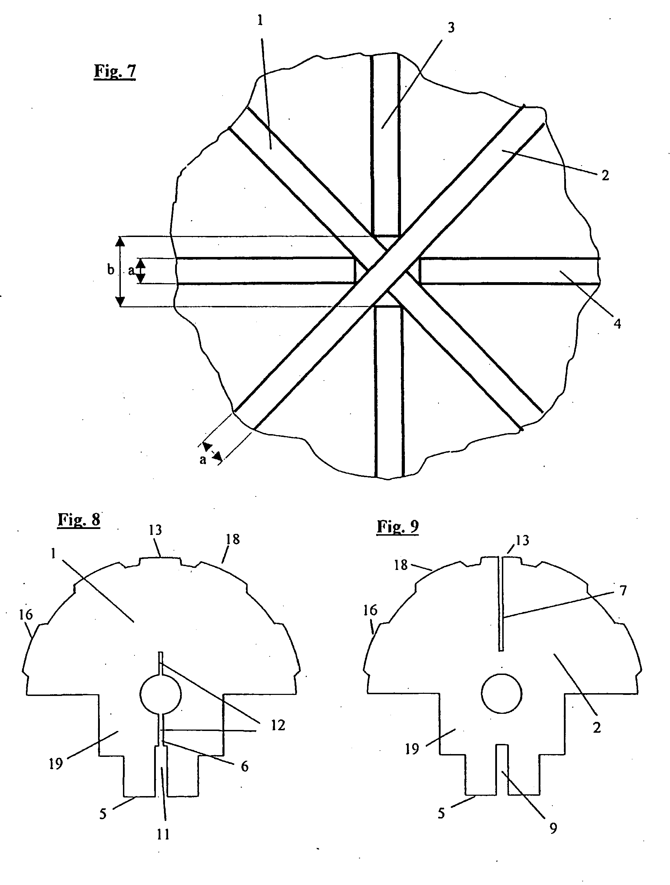

Chaque lame a un axe de symétrie longitudinal qui coïncide avec l'axe de rotation 20 de la fraise. Le tranchant des lames, c'est-à-dire le fil 18, est ici de forme sensiblement semi-circulaire. Cette forme est particulièrement bien adaptée aux fraises à cotyle. Il est cependant évident que de nombreuses autres formes peuvent être utilisées, selon les buts auxquels la fraise est destinée. Chaque lame présente au moins une fente 6, 7, 8, 9 ou 10, qui est sur l'axe de rotation 20. Cette fente longitudinale permet l'insertion de lames les unes dans les autres. Chaque lame présente également une partie inférieure 19, qui a ici la forme d'un rectangle ou d'un double rectangle qui prolonge la lame en direction de l'arbre rotatif d'un outil, non représenté ici, auquel la fraise doit être fixée. C'est cette partie inférieure 19 qui permet ce raccordement. On voit à la figure 1 que la fente 6 est pratiquée dans la première lame 1 du bord 5 de la partie inférieure 19 jusqu'au milieu de la hauteur de la lame. A l'inverse, la fente 7 de la deuxième lame 2 est ménagée depuis la mi-hauteur jusqu'au sommet 13 de la lame. De la sorte, les deux premières lames s'emboîtent l'une dans l'autre à angle droit. Quant aux deux lames suivantes, elles présentent des fentes 8, 9 et 10 de forme et de disposition plus complexes. On note d'abord que, à la figure 1, les parties inférieures 19 de ces deux lames suivantes sont plus longues que celles des deux premières. En effet, les troisième et quatrième lames présentent chacune respectivement une fente 8 et 10 qui va de leur sommet 13 jusque dans leur partie inférieure 19. De plus, ces fentes 8 et 10 de la troisième et de la quatrième deux lames sont plus larges que celles que présentent les deux premières lames. La troisième lame 3 présente d'autre part une fente inférieure 9 qui va de son bord inférieur 5 jusqu'au quart de ladite partie inférieure ; cette fente inférieure 9 est plus étroite que la fente supérieure 8. La fente 10 de la quatrième lame présente deux secteurs 11 et 12 ; le secteur extérieur 11, qui va du sommet 13 de la quatrième lame jusque vers le milieu de la hauteur de la partie inférieure 19, est plus large que le secteur intérieur 12. La largeur de la fente inférieure 9 de la troisième lame est la même que celle du secteur intérieur 12 de la fente 10 de la quatrième lame correspond à l'épaisseur de la lame que l'on y insère. La longueur de la fente 8 de la troisième lame et celle du secteur extérieur 11 de la quatrième lame est la même et correspond à la hauteur totale de chacune des deux premières lames.
La troisième lame 3 et la quatrième lame 4 sont jointes l'une à l'autre par la fente inférieure 9 et par le secteur intérieur 12, à angle droit.
Le groupe formé par les deux lames 1 et 2 est ensuite inséré dans la fente supérieure 8 et le secteur 11 du groupe formé par la troisième et la quatrième lames. La nécessité de la largeur accrue de la fente supérieure 8 et du secteur extérieur 11 pour l'insertion des deux premières lames est mise en évidence dans les figures 4 et 7. La figure 7 montre que la largeur des fentes 6 et 7 doit correspondre à l'épaisseur « a » pour les deux lames 1 et 2 qui se croisent en « X » dans ce dessin.

Quant aux deux lames qui se croisent en « + » (et qui correspondent aux deux lames 3 et 4), elles présentent respectivement une fente supérieure 8 et un secteur 11 de largeur « b », qui permet l'insertion du groupe formé par les deux lames de gauche. On voit ici que la largeur « b » est nettement supérieure à la largeur « a » (selon Pythagore, $b = a(1+\sqrt{2})$). Un ajustement précis des largeurs a et b des fentes et des lames permet d'éviter tout mouvement relatif des lames les unes par rapport aux autre et d'obtenir un ensemble rigide. Une fois les quatre lames réunies entre elles, leurs parties inférieures 19 sont par exemple chassées dans une douille 14, qui est concentrique à l'axe de rotation 20 de la fraise. Ainsi fixée sur la périphérie de leurs parties inférieures, les lames forent un ensemble très rigide. La présence d'une telle douille 14 n'est cependant pas indispensable dans tous les cas : dans plusieurs cas d'utilisation de la fraise, une rigidité suffisante est déjà assurée par un ajustement correct des largeurs a et b des fentes.

Il est également possible de fixer les lames réunies dans la douille par soudure, par collage ou de toute autre manière. Le chassage reste le procédé le plus simple. De même, la douille peut prendre une autre forme que celle d'un cylindre, la forme cylindrique restant la plus rationnelle.

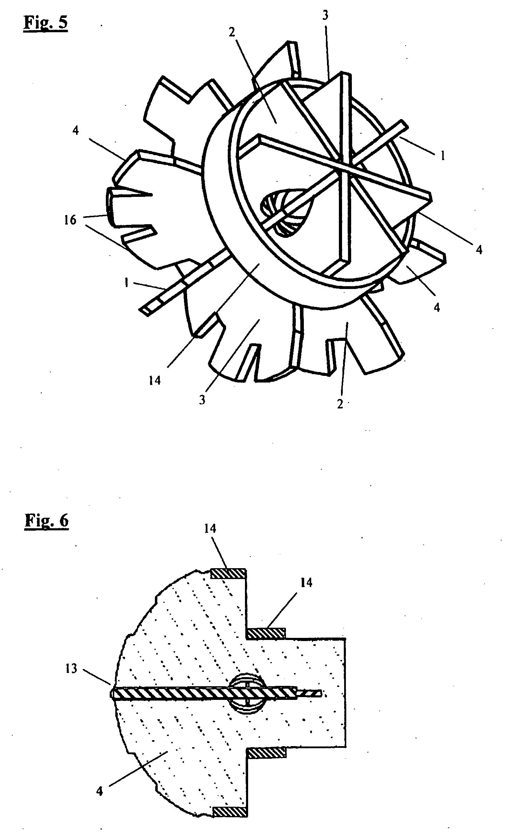

On peut rendre cet ensemble encore plus rigide, si nécessaire, en y ajoutant une seconde douille 14, de plus grand diamètre, et qui enserre les lames à une plus grande distance de l'axe de rotation 20. Cette forme d'exécution particulière est montrée à la figure 6. D'autre part, il est aussi possible de placer la douille 14 seulement sur la périphérie de la fraise, sans qu'une autre douille enserre l'ensemble au niveau des parties inférieures 19 des lames.

Dans une forme d'exécution représentée aux figures 1 à 3, la douille 14 sert non seulement à fixer solidement entre elles les lames 1, mais également à fixer longitudinalement, c'est-à-dire dans la direction de l'axe de rotation 20, la fraise à l'arbre rotatif qui la meut. La douille 14 présente ainsi, dans sa partie opposée à celle qui reçoit les parties inférieure 19 des lames, plusieurs fentes 22 longitudinales. Ces fentes 22 assouplissent le bas de la douille et permettent d'encliqueter aisément la douille sur un arbre rotatif et de l'y fixer grâce au cran 23 montré à la figure 3. Ce mode de fixation est cependant loin d'être le seul possible.

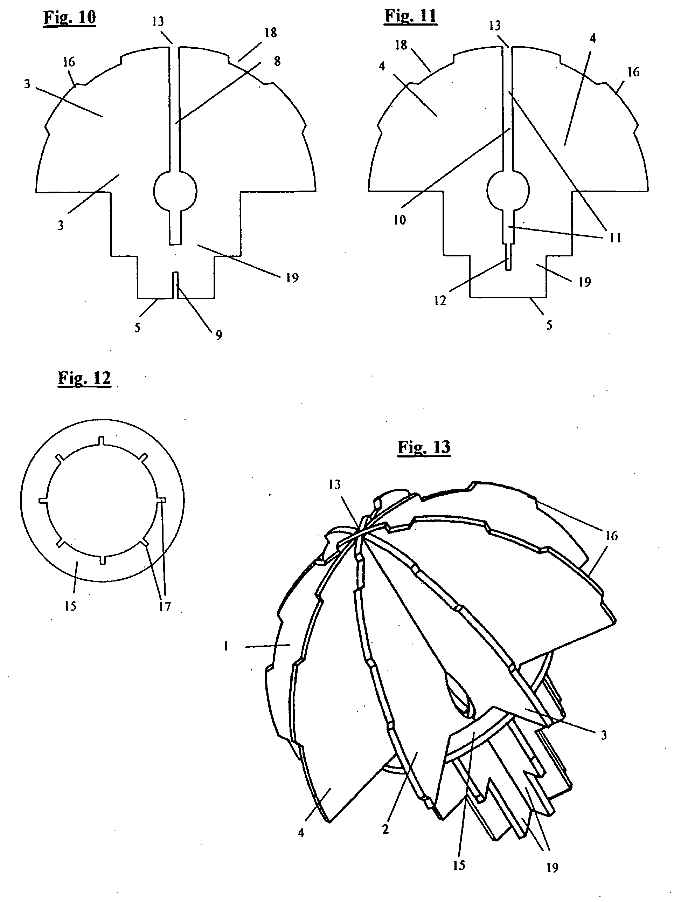

Une autre manière de rendre rigide l'ensemble formé par les lames consiste à remplacer la douille par une plaque, de préférence en forme d'anneau plat 15, qui présente des encoches 17 dans lesquelles viennent s'insérer les lames. La figure 12 montre un tel anneau, et la figure 13 le montre monté autour des parties inférieures 19 des lames assemblées.

Les lames 1 ont pour axe de symétrie l'axe de rotation 20 de la fraise. Afin de permettre leur affûtage, si nécessaire, il est avantageux de prévoir un trou 21 placé sur cet axe de rotation.

Dans les dessins, les lames ont une arête tranchante 18 semi-circulaire présentant des dents 16, qui peuvent être utiles pour faciliter le fraisage en fonction des buts visés. Comme on l'a vu plus haut, de préférence et dans la mesure du possible, les dents sont taillées et leur inclinaison est obtenue dans une seule et même opération d'étampage.

On a vu que, dans la forme d'exécution représentée dans les figures 1 à 3, la hauteur des deux premières lames 1 et 2 était inférieure à celle des troisième et quatrième lames 3 et 4. Or, il peut être avantageux de disposer d'une hauteur égale de chaque lame, notamment en vue de la fixation de la fraise sur un outil.

Dans une forme préférée de d'exécution de l'invention, la première lame 1, telle que représentée à la figure 8, présente une fente 6 comprenant deux secteurs : un secteur extérieur 11, qui part du bord inférieure 5 de la lame, et un secteur intérieur 12, qui continue le secteur extérieur en direction du sommet 13, et qui est plus étroit que le secteur extérieur. De même, comme le montre la figure 9, la deuxième lame 2 présente, outre la fente supérieure 7 qui part du sommet 13 vers le bas, une fente inférieure 9 qui part du bord inférieur 5 vers le haut.

Le secteur extérieur 11 de la première lame 1 et la fente inférieure 9 de la deuxième lame 2 sont plus larges respectivement que le secteur intérieur 12 et que la fente supérieure 7. Cette largeur permet d'allonger la partie inférieure 19 pour arriver à la même hauteur que dans les troisième et quatrième lames 3 et 4, qui sont représentée aux figures 10 et 11. La largeur accrue du secteur extérieur 12 et de la fente inférieure 9 donne en effet suffisamment de place pour que s'y loge l'ensemble formé par la troisième et la quatrième lames assemblées.

La minceur des lames, découpées dans une feuille métallique, et leur position dans le plan de l'axe de rotation, permettent à l'utilisateur de voir la portion à fraiser, même en cours d'opération, entre les lames.

La présence de quatre lames au lieu de deux seulement est garante d'une rigidité suffisante pour effectuer des opérations sans plus de risques qu'avec une fraise classique.

La fabrication des lames et leur assemblage sont aisés et bon marché, ce qui permet de les jeter au lieu de procéder à leur nettoyage, opération onéreuse.

Possibilités d'application industrielle

La fraise et les lames objets de l'invention son utilisables essentiellement dans les opérations permettant de placer des prothèses de la hanche.

**Revendications**

1. Fraise chirurgicale formée de plusieurs lames plates assemblées entre elles au moyen de fentes (6, 7, 8, 9, 10) dont l'axe de symétrie longitudinal coïncide avec l'axe de rotation (20) de la fraise, **caractérisée en ce qu'**elle comprend au moins quatre lames (1, 2, 3, 4) et **en ce qu'**au moins une des fentes et/ou secteurs de la ou desdites fentes ont une largeur différente de celle d'au moins une autre fente et/ou secteur de fente.

**2.** Fraise selon la revendication 1, **caractérisée en ce qu'**elle comprend quatre lames (1, 2, 3, 4), dont la première (1) présente au moins une fente (6) qui part du bord inférieur (5) de la lame, dont la deuxième lame (2) présente au moins une fente (7) partant du sommet (13) de la lame, chacune desdites fentes (6, 7) ayant, au moins sur un secteur, une largeur correspondant à l'épaisseur de la lame dans laquelle est ménagée l'autre fente, dont la troisième lame (3) présente au moins une fente supérieure (8) partant du sommet (13) de ladite troisième lame et au moins une fente inférieure (9) partant du bord inférieur (5) de ladite troisième lame, la largeur de ladite fente supérieure (8) étant plus grande que la largeur de ladite fente inférieure (9), et dont la quatrième lame (4) présente au moins une fente (10) partant du sommet (13) de ladite lame, ladite fente (10) comprenant au moins un secteur extérieur (11) et un secteur intérieur (12), la largeur dudit secteur extérieur (11) étant plus grande que la largeur dudit secteur intérieur (12), la largeur de la fente inférieure (9) de la troisième lame (3) correspondant à l'épaisseur de la quatrième lame (4), la largeur du secteur intérieur (12) de la fente (10) de la quatrième lame (4) correspondant à l'épaisseur de la troisième lame (3), et la largeur de la fente supérieure (8) de la troisième lame (3) et la largeur du secteur extérieur (11) de la quatrième lame (4) étant les mêmes et telles que, une fois emboîtées l'une dans l'autre, les deux premières lames puissent être insérées dans ladite fente supérieure (8) de la troisième lame (3) et dans le secteur extérieur (11) de la quatrième lame (4), lesdites troisième et quatrième lames étant elles-mêmes emboîtées l'une dans l'autre.

**3.** Fraise selon la revendication 1 ou 2, **caractérisée en ce que** la fente (6) de la première lame (1) présente au moins deux secteurs (11, 12) de largeur différente, le secteur extérieur (11) étant plus large que le secteur intérieur (12).

**4.** Fraise selon l'une des revendications 1 à 3, **caractérisée en ce que** la deuxième lame (2) présente au moins une fente inférieure (9) partant de son bord inférieur (5), ladite fente inférieure étant plus large que la fente (7) qui part du sommet (13) de la lame.

**5.** Fraise selon l'une des revendications 1 à 4, **caractérisée en ce qu'**elle comprend au moins un élément raidisseur (14, 15) qui encercle partiellement et tient ensemble les lames composant la fraise.

**6.** Fraise selon la revendications 5, **caractérisée en ce que** l'élément raidisseur est une douille cylindrique (15).

**7.** Fraise selon la revendication 5, **caractérisée en ce** l'élément raidisseur est un anneau plat (15) présentant des encoches (17) dans lesquelles viennent s'insérer les lames.

**Claims**

**1.** Surgical reamer formed from several flat blades assembled together by means of slots (6, 7, 8, 9, 10) wherein the longitudinal axis of symmetry is coincident with the axis of rotation (20) of the reamer, **characterised in that** it comnprises at least four blades (1, 2, 3, 4) and that at least one of the slots and/or sections of said slot or slots has a different width from that of at least one other slot and/or section of a slot.

**2.** Reamer according to claim 1, **charcterised in that** it comprises four blades (1, 2, 3, 4), the first of which (1) has at least one slot (6) which runs from the lower edge (5) of the blade, of which the second blade (2) has at least one slot (7) running from the top (13) of the blade, each of said slots (6, 7) having, at least in one section, a width corresponding to the thickness of the blade into which is fitted the other slot, in which the third blade (3) has at least one upper slot (8) running from the top (13) of said third blade and at least one lower slot (9) running from the lower edge (5) of said third blade, the width of said upper slot (8) being greater than the width of said lower slot (9), and of which the fourth blade (4) has at least one slot (10) running from the top (13) of said blade, said slot (10) comprising at least one outer section (11) and one inner section (12), the width of said outer section (11) being greater than the width of said inner section (12), the width of the lower slot (9) of the third blade (3) corresponding to the thickness of the fourth blade (4), the width of the inner section (12) of the slot (10) of the fourth blade (4) corresponding to the thickness of the third blade (3), and the width of the upper slot (8) of the third blade (3) and the outer section (11) of the fourth blade (4) being the same and such that, once fitted together, the first two blades can be inserted into said upper slot (8) of the third blade (3) and into the outer section (11) of the fourth blade (4), said third and fourth blades being themselves fitted together.

**3.** Reamer according to claim 1 or 2, **characterised in that** the slot (6) of the first blade (1) has at least two sections (11, 12) of different widths, the outer section (11) being wider than the inner section (12).

**4.** Reamer according to one of the claims 1 to 3, **characterised in that** the second blade (2) has at least one lower slot (9) running from its lower edge (5), said lower slot being wider than the slot (7) which runs from the top (13) of the blade.

**5.** Reamer according to one of the claims 1 to 4, **characterised in that** it comprises at least one rigidifying element (14, 15) which partially encircles and holds together the blades making up the reamer.

**6.** Reamer according to claim 5, **characterised in that** the rigidifying element is a cylindrical bushing (15).

**7.** Reamer according to claim 5, **characterised in that** the rigidifying element is a flat ring (15) with notches (17) into which the blades are inserted.

**Patentansprüche**

**1.** Chirurgischer Fräser gebildet aus mehreren flachen Messern, die miteinander mit Hilfe von Schlitzen (6, 7, 8, 9, 10) verbunden sind, deren Längssymmetrieachsen mit der Rotationsachse (20) des Fräsers zusammenfallen, **dadurch gekennzeichnet, dass** er wenigstens vier Messer (1, 2, 3, 4) aufweist und dass wenigstens einer der Schlitze und/oder einer der Abschnitte des Schlitzes oder der Schlitze eine andere Breite als wenigstens ein anderer Schlitz und/oder Schlitzabschnitt aufweist.

**2.** Fräser nach Anspruch 1, **dadurch gekennzeichnet, dass** er vier Messer (1, 2, 3, 4) aufweist, von denen das erste (1) wenigstens einen Schlitz (6) aufweist, der von der Unterkante (5) des Messers ausgeht, wobei das zweite Messer (2) wenigstens einen von dem Scheitel (13) des Messers ausgehenden Schlitz (7) aufweist, wobei jeder der genannten Schlitze (6, 7) wenigstens in einem Abschnitt eine Breite entsprechend der Dicke des Messers aufweist, in welchem der andere Schlitz vorgesehen ist, wobei das dritte Messer (3) wenigstens einen von dem Scheitel (13) des dritten Messers ausgehenden oberen Schlitz (8) und wenigstens einen von der Unterkante (5) des dritten Messers ausgehenden unteren Schlitz (9) aufweist, wobei die Breite des oberen Schlitzes (8) größer als die Breite des unteren Schlitzes (9) ist, und wobei das vierte Messer (4) wenigstens einen von dem Scheitel (13) des Messers ausgehenden Schlitz (10) aufweist, wobei der genannte Schlitz (10) wenigstens eine äußeren Abschnitt (11) und einen inneren Abschnitt (12) aufweist, wobei die Breite des äußeren Abschnitts (11) größer als die Breite des inneren Abschnitts (12) ist, wobei die Breite des unteren Schlitzes (9) des dritten Messers (3) der Dicke des vierten Messers (4) entspricht, die Breite des inneren Abschnitts (12) des Schlitzes (10) des vierten Messers (4) der Dicke des dritten Messers (3) entspricht und die Breite des oberen Schlitzes (8) des dritten Messers (3) und die Breite des äußeren Abschnitts (11) des vierten Messers (4) gleich sind und derart ausgebildet sind, dass, wenn sie ineinander gesteckt sind, die beiden ersten Messer in den oberen Schlitz (8) des dritten Messers (3) und in den äußeren Abschnitt (11) des vierten Messers (4) eingesetzt werden können, wobei das dritte und vierte Messer selbst ineinander gesteckt sind.

**3.** Fräser nach Anspruch 1 oder 2, **dadurch gekennzeichnet, dass** der Schlitz (6) des ersten Messers (1) wenigstens zwei Abschnitte (11, 12) unterschiedlicher Breite aufweist, wobei der äußere Abschnitt (11) breiter als der innere Abschnitt (12) ist.

**4.** Fräser nach einem der Ansprüche 1 bis 3, **dadurch gekennzeichnet, dass** das zweite Messer (2) wenigstens einen unteren Schlitz (9) aufweist, der von seiner Unterkante (5) ausgeht, wobei der untere Schlitz breiter als der von dem Scheitel (13) des Messers ausgehende Schlitz (7) ist.

**5.** Fräser nach einem der Ansprüche 1 bis 4, **dadurch gekennzeichnet, dass** er wenigstens ein Versteifungselement (14, 15) aufweist, das die den Fräser bildenden Messer teilweise einschließt und zusammenhält.

**6.** Fräser nach Anspruch 5, **dadurch gekennzeichnet, dass** das Versteifungselement eine zylindrische Hülse (15) ist.

**7.** Fräser nach Anspruch 5, **dadurch gekennzeichnet, dass** das Versteifungselement ein flacher Ring (15) ist, der Einschnitte (17) aufweist, in welche die Messer eingesetzt werden.

**Fig. 1**

**Fig. 2**

**Fig. 3**

**Fig. 4**

**Fig. 5**

**Fig. 6**

**Fig. 7**

**Fig. 8**

**Fig. 9**

11

**Fig. 10**

**Fig. 11**

**Fig. 12**

**Fig. 13**

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CH 692600
- CH 690021
- FR 2281095
- US 4811632 A
- US 5100267 A
- FR 1041311
- FR 1031888
- US 3702611 A
- US 4621637 A
- WO 9831291 A
- WO 0249516 A